# EUROPEAN PATENT APPLICATION

(11) **EP 1 335 022 A1**
(43) Date of publication of application: **13.08.2003**
(21) Application number: 01967826.7
(22) Date of filing: 25.09.2001
(51) Int. Cl.: C12N 15/29, C12N 15/09, C12N 5/10, C07K 14/415, C12P 21/02, A01H 5/00

(54) **MODIFICATION OF PLANT CELL WALL COMPONENT AND METHOD OF REGULATING DEVELOPMENT DIFFERENTIATION**

(30) Priority: 25.09.2000 JP 2000291072
(71) Applicant: Oji Paper Co., Ltd., Tokyo 104-0061 (JP)
(72) Inventor: TOMITA, Keiji, Suzuka-shi, Mie 513-0844 (JP); SATO, Shigeru, Suzuka-shi, Mie 513-0809 (JP); MIYASHITA, Kyoko, Nara-shi, Nara 631-0076 (JP); SHIBATA, Daisuke, Kisarazu-shi, Chiba 292-0814 (JP); KATO, Tomohiko, Kisarazu-shi, Chiba 292-0801 (JP)
(74) Representative: Callon de Lamarck, Jean-Robert
(86) International application number: JP0108301
(87) International publication number: WO02024914

(57) **Abstract**

By using a positional cloning technique, a single gene causing the ACW2 mutation, which results in abnormalities in plant cell wall synthesis and development/differentiation, has been successfully identified over a broad chromosomal region and isolated. Since the *ACW2* gene thus isolated affects plant cell wall synthesis and development/differentiation, the present inventors discovered that the plant cell wall can be modified or the development/differentiation can be regulated by targeting the *ACW2* gene, or genes analogous thereto.

## Description

### Technical Field

The present invention relates to the modification of cell wall components and regulation of development/differentiation in plants, as well as molecules used for that purpose.

### Background Art

The regulation of plant cell wall biosynthesis has various important significances in the fields of industry and agriculture. For example, by elevating cellulose/hemicellulose contents, the modification of plant cell wall components can lead to the production of plants that can supply fiber raw materials such as pulp and the improvement of the digestion/absorption efficiency of useful farm and feed crops. Thus, such modifications are significant from the aspects economical efficiency and profitability. Furthermore, structural modification of polysaccharides, which are cell wall components, can lead to the production of raw material plants having novel industrial values.

The synthesis of the cell wall and polysaccharide components analogous thereto is conduoted not only in bacteria, fungi, and plants, but also in animals. Mechanisms of cell wall synthesis have been actively analyzed on the molecular level in spite of their industrial importance. Recently, molecular-level studies on plant cell wall synthesis are starting to be conducted using analytical techniques of molecular genetics. Until now, cellulose synthase, and such, have been reported as genes involved in cell wall synthesis (T. Arioli et al., "Molecular analysis of cellulose biosynthesis in *Arabidopsis*", Science 279: 717-720 (1998)). However, it is thought that many genes involved in cell wall synthesis are yet to be isolated, and that there are still many unknown mechanisms relating to cell wall synthesis (references: Y. Kawagoe and D. P. Delmer, "Pathways and genes involved in cellulose biosynthesis", Genetic engineering 19, Plenum Press, New York (1997); K. Nishitani, "Construction and Restructuring of the cellulose-xyloglucan framework in the apoplast as mediated by the xyloglucan-related protein family -A hypothetical scheme", J. Plant Res. 111: 159-166 (1998)).

In cell division, it is though that first Golgi body-derived vesicles fuse together near the cell division surface to form phragmoplasts, and then callose accumulate therein to make a cell plate. Several proteins present in phragmoplasts and cell plates have been reported (M. Heese et al., Current Opinion in Plant Biology 1: 486-491 (1998); X. Gu and D. P. S. Verma, EMBO J. 15: 695-704 (1996)). Phragmoplastin is one of these proteins, termed the "dynamin-like protein" in general, and known to be broadly present in the whole plant and animal world (X. Gu and D. P. S. Verma, Plant Cell 9: 157-169 (1997); D. Otsuga, et al., J. Cell Biol. 143, 333-349 (1998); S. G. Kang et al., Plant Mol. Biol. 38: 437-447 (1998); D. C. Wienke et al., Molecular Biology of the Cell 10: 225-243 (1999)). However, the function of this protein has not been elucidated yet.

In connection with this, in a group of *Arabidopsis thaliana* treated with a chemical mutagen EMS, a mutant (tagged with the accession number #55) having abnormalities in cell and organ morphologies as well as in cell wall synthesis has been found. This mutation was designated "altered cell wall (acw) 2", and the gene causing this mutation was named the "*ACW2*" gene. Histochemical analysis using electron microscopes has indicated the involvement of the *ACW2* gene in cell wall biosynthesis (S. Sato, T. Kato, K. Takabe, andD. Shibata., "Analysis of a temperature-sensitive mutant of *Arabidopsis* involved in cell wall synthesis", Annual Meeting of the Japanese Plant Physiological Society, at Hokkaido University, May 3 (1998)).

However, the *ACW2* gene has neither been isolated nor identified.

### Disclosure of the Invention

It is an objective of the present invention to provide the *ACW2* gene, genes analogous thereto, and proteins encoded by these genes. It is also another objective of this invention to regulate cell wall biosynthesis and development/differentiation of plants using these genes.

The present inventors conducted exhaustive studies to solve the above-described problems, and using a positional cloning technique, succeeded in identifying over a broad chromosomal region a single gene causing the ACW2 mutation that results in abnormalities in cell wall biosynthesis and development/differentiation of plants, and isolating this gene. Furthermore, based on the effect of the *ACW2* gene thus isolated on cell wall biosynthesis and development/differentiation of plants, the present inventors discovered that it is possible to improve the cell wall and regulate development/differentiation of plants by targeting the *ACW2* gene, and genes analogous thereto.

That is, the present invention relates to the *ACW2* gene involved in plant cell division, genes analogous thereto, and proteins encoded by these genes, as well as the improvement of cell wall components and regulation of development/differentiation of plants by targeting these genes. More specifically, the present invention provides the following:
(1) A DNA of any one of (a) to (d), wherein said DNA is used to modify a plant cell wall component or to regulate development/differentiation of a plant:
   (a) a DNA encoding a protein comprising the amino acid sequence of SEQ ID NO: 1,
   (b) a DNA comprising the nucleotide sequence of SEQ ID NO: 2,
   (c) a DNA encoding a protein comprising the amino acid sequence of SEQ ID NO: 1 in which one or more amino acids have been substituted, deleted, or added, wherein said protein is functionally equivalent to the protein comprising the amino acid sequence of SEQ ID NO: 1, and
   (d) a DNA that (i) is capable of hybridizing to the DNA comprising the nucleotide sequence of SEQ ID NO: 2 under stringent conditions, and (ii) encodes a protein that is functionally equivalent to the protein comprising the amino acid sequence of SEQ ID NO: 1;
(2) A DNA coding for anti-sense RNA complementary to the transcriptional product of the DNA of (1), wherein said DNA is used for modifying a plant cell wall component or for regulating plant development/differentiation;
(3) A DNA coding for RNA having a ribozyme activity to specifically cleave a transcriptional product of the DNA of (1), wherein said DNA is used for modifying a plant cell wall component or for regulating plant development/differentiation;
(4) A DNA used for modifying a plant cell wall component or regulating plant development/differentiation, wherein said DNA (i)encodes an RNA that suppresses the expression of the DNA of (1) by co-suppression when it is expressed in plant cells, and (ii) has 90% or more homology to the DNA of (1);
(5) A DNA used for modifying a plant cell wall component or regulating development/differentiation, wherein said DNA encodes a protein having a dominant negative characteristic against a plant cell endogenous protein encoded by the DNA of (1);
(6) A vector comprising the DNA according to any one of (1) to (5);
(7) A transformed cell retaining a DNA according to any one of (1) to (5), or the vector according to (6);
(8) A protein encoded by the DNA according to (1);
(9) A method for preparing the protein according to (8), comprising the steps of culturing transformed cells retaining the DNA according to (1), or a vector containing said DNA, and recovering the protein expressed from said transformed cells or culture supernatant thereof;
(10) A transformed plant cell retaining the DNA according to any one of (1) to (5), or the vector according to (6);
(11) A transformed plant comprising the transformed plant cell according to (10);
(12) A transformed plant which is a progeny or a clone of the transformed plant according to (11);
(13) The transformed plant according to (11) or (12), wherein a cell wall component has been modified;
(14) The transformed plant according to (13), wherein the modification of the cell wall component is the alteration of the glucan content;
(15) The transformed plant according to (13), wherein the modification of the cell wall component is the alteration of the cellulose content;
(16) The transformed plant according to (11) or (12), wherein the development/differentiation has been modified;
(17) The transformed plant according to (16), wherein the modification of development/differentiation is the alteration of cell division;
(18) The transformed plant according to (16), wherein the modification of development/differentiation is the alteration of the number and size of cells;
(19) A propagation material of the transformed plant according to any one of (11) to (18);
(20) An antibody binding to the protein according to (8); and
(21) An agent for modifying a plant cell wall component or regulating plant development/differentiation including the DNA according to any one of (1) to (5), the vector according to (6), the protein according to (8), or the antibody according to (20).

The present invention provides the "ACW2" protein involved in plant cell division. The nucleotide sequence of the cDNA of the *ACW2* gene, which was isolated by the inventors using a positional cloning technique as the causative gene of the acw2 mutation that results in abnormalities in cell wall biosynthesis and development/differentiation of *Arabidopsis thaliana*, is shown in SEQ ID NO: 2. The nucleotide sequence of the genomic DNA is shown in SEQ ID NO: 3, and the amino acid sequence of the ACW2 protein encoded by these genes is shown in SEQ ID NO: 1.

The acw2 mutation which is known to result in a phenotype with an alteratered cell wall component and aberrant cell division in plant cell wall biosynthesis and development/differentiation, was found to be due to a substitution of the nucleotide guanine at the 571^{st} position in the *ACW2* gene isolated by the inventors with adenine, and thereby, a replacement of the amino acid aspartic acid at the 191^{st} position in the ACW2 protein with asparagine (Example 1). These facts prove that the *ACW2* gene isolated by the inventors functions in plant cell division, and that its mutation leads to aberrations in cell wall biosynthesis and development/differentiation of plants. Such a close relationship between the *ACW2* gene and plant cell wall biosynthesis as well as development/differentiation, shows the possibility of regulating plant cell wall biosynthesis and development/differentiation by regulating *ACW2* gene expression. Therefore, the "ACW2" protein and "*ACW2*" gene can be used to regulate plant cell wall biosynthesis and development/differentiation, or as targets for the regulation.

Furthermore, the present invention provides a protein functionally equivalent to the "ACW2" protein. Herein, "functionally equivalent" means that the protein functions in plant cell wall biosynthesis and development/differentiation. Whether or not a protein functions in plant cell wall biosynthesis and development/differentiation can be determined by functional complementation testing based on the expression of the protein in mutants. Alternatively, the protein function may be inhibited *in vivo* and the resulting alterations in cell wall biosynthesis and development/differentiation (changes in cell wall components and organ/cell morphologies) may be detected.

As an embodiment of a method for isolating such a functionally equivalent protein, the method of introducing a mutation into an amino acid in the protein is well known to those skilled in the art. It is possible for those skilled in the art to appropriately substitute amino acid residue(s) in the natural "ACW2" protein (e.g. the protein set forth in SEQ ID NO: 1) so as to prepare a modified protein functionally equivalent thereto using a method known in the art. Moreover, amino acid mutations may also occur spontaneously. Proteins of the present invention include those having the amino acid sequence of the natural "ACW2" protein in which one or more amino acids have been substituted, deleted, or added, while retaining a function equivalent to that of the natural protein. Modification of amino acids in proteins is usually in the range of not more than 50 residues in the whole number of amino acids, preferably not more than 30, more preferably not more than 10, and even more preferably, not more than 3 amino acid residues. Amino acid modifications may be performed, for example, in the case of mutations and substitutions, using a "Transformer Site-directed Mutagenesis Kit" or "ExSite PCR-Based Site-directed Mutagenesis Kit" (Clontech) , and, in the case of deletions, using a "Quantum leap Nested Deletion Kit" (Clontech) and the like.

Furthermore, examples of other embodiments of methods for isolating functionally equivalent proteins are the hybridization method (Southern, J. Mol. Biol. 98: 503 (1975); Maniatis et al., "Molecular Cloning", Cold Spring Harbor Laboratory Press) and PCR technique (H. A. Erlich (ed.) PCR Technology, Stockton Press, New York (1989)). That is, those skilled in the art can routinely isolate DNA highly homologous to the *ACW2* gene using the nucleotide sequence of *ACW2* gene (SEQ ID NO: 2) or a portion thereof as a probe, or employing oligonucleotides which hybridize to the nucleotide sequence of *ACW2* gene (SEQ ID NO: 2) or a portion thereof as primers so as to obtain proteins functionally equivalent to the ACW2 protein from the DNA. Proteins of this invention also include those proteins that are functionally equivalent to the "ACW2" protein encoded by the DNAs thus isolated 'through the hybridization and PCR techniques.

Hybridization for isolating genes functionally equivalent to *ACW2* can be carried out under conditions in which, after hybridization at 65°C, washings in 2x SSC (3 M NaCl 0.3 M sodium citrate) containing 0.1% SDS or 2x SSPE (3.6 M NaCl, 0.2 M sodium phosphate (pH 7.7), 0.02 M Na₂-EDTA) at 65°C for 10 min are repeated three times in total. In a more stringent hybridization, after the hybridization at 65°C, washings may be performed once in 2x SSC or 2x SSPE containing 0.1% SDS at 65°C for 10 min, and then twice in 1x SSC or 1x SSPE containing 0.1% SDS at 65°C for 10 min. In an even more stringent hybridization, after the hybridization at 65°C, washings may be performed once each at 65°C for 10 min in 2x SSC or 2x SSPE containing 0.1% SDS, then in 1x SSC or 1x SSPE containing 0.1% SDS, and further in 0.1x SSC or 0.1x SSPE containing 0.1% SDS. As for the hybridization solution, the one described in "Molecular Cloning (Maniatis, T. et al., Cold Spring Harbor Laboratory Press)" and the like may be used.

From the functional point of view, proteins encoded by DNAs obtained by the hybridization and PCR techniques have preferably 45% or more, more preferably 60% or more, more preferably 75% or more, even more preferably 90% or more, and further more preferably 95% or more homology to the "ACW2" protein in the amino acid sequence. Sequence homology can be determined by a homology search using, for example, a gene sequence analysis software, GENETYX (Software Development Co.) , Blast search (see the BLAST website of the National Center for Biotechnology Information), and the like. In the Blast search, the programs of BLASTn (for the nucleic acid content) and BLASTx (for the amino acid content) (Altschul et al., J. Mol. Biol. 215: 403-410 (1990)), which are based on the algorithm BLAST by Karlin and Altschul (Proc. Natl. Acad. Sci. U.S.A. 87: 2264-2268 (1990); Proc. Natl. Acad. Sci. U.S.A. 90: 5873-5877 (1993)) can be used. When analyzing a nucleic acid sequence with BLASTn, parameters are set at, for example, score = 100, and word length = 12. When analyzing an amino acid sequence with BLASTx, parameters are set at, for example, score = 50 and word length = 3. When an amino acid sequence is analyzed using the Gapped BLAST program, the analysis is carried out as described by Altschul et al. (Nucleic. Acids. Res. 25: 3389-3402 (1997)). When using both BLAST and Gapped BLAST programs, the default parameters of the respective programs are used. Specific techniques of these analytical methods are well known.

There is no particular limitation on the type of other plants that can be used for isolating such proteins, and examples are rice, cotton, eucalyptus, poplar, etc.

The proteins of the present invention can be prepared, besides as natural proteins, as recombinant proteins formed using gene recombination techniques according to methods known to those skilled in the art. A natural protein can be prepared, for example, by immunizing small animals such as rabbits with a recombinant protein prepared by the method described below to obtain an antibody, and binding the antibody obtained to a suitable adsorbent (such as CNBr- or tosyl-activatedagarose) to prepare a column, and purifying a protein extract of rice leaves using the column. On the other hand, it is possible to prepare a recombinant protein according to standard methods, for example, by inserting DNA coding for a protein of the present invention into an appropriate expression vector, introducing the vector into suitable cells, and purifying the recombinant protein from the transformed cells.

Examples of cells used for the production of recombinant proteins are plant cells, *Escherichia coli*, yeasts, animal cells, insect cells and the like. Examples of vectors for expressing a recombinant protein in cells are the plasmids "pBI121" and "pBI101" (Clontech) for plant and yeast cells, plasmids "pET Expression system" (Stratagene) and "GST gene fusion Vectors" (Pharmacia) for *Escherichia coli*, plasmid "pMAM" (Clontech) for mammalian cells, plasmid "pBacPAK8.9" (Clontech) for insect cells, etc. Insertion of DNA into vectors can be carried out by standard methods such as those described in "Molecular Cloning" (Maniatis et al., Cold Spring Harbor Laboratory Press) . Vectors may be introduced into host cells by standard methods such as the electroporation method, microinjection method, particle gun method, or the like according to the host cell used.

The purification of a recombinant protein of the present invention from the transformed cells thus obtained may be carried out, depending on the properties of the protein, by a suitable combination of precipitation using salting-out and organic solvents, ion-exchange chromatography, affinity chromatography, column chromatography with immunoadsorbents, gel filtration, SDS electrophoresis, isoelectric focusing, and the like. Furthermore, when the recombinant protein of the present invention is expressed as a fusion protein with a label such as glutathione-S-transferase, the protein may be purified by affinity chromatography for the label, etc.

The present invention also provides DNAs encoding the above-described proteins of the present invention. There is no particular limitation on the type of DNAs of this invention as long as they are capable of encoding the proteins of this invention, and include genomic DNA, cDNA, chemically synthesized DNA, etc. Genomic DNAs may be prepared by conducting PCR (Saiki et al., Science 239: 487 (1988)) with genomic DNA prepared according to a method described in literature (Rogers and Bendich, Plant Mol. Biol. 5: 69 (1985)) as a template using primers prepared based on a nucleotide sequence of a DNA of this invention (e.g. the nucleotide sequence set forth in SEQ ID NO: 2). Furthermore, in the case of cDNA, it may be prepared, according to the standard method (Maniatis et al., "Molecular Cloning" , Cold Spring Harbor Laboratory Press) , by preparing mRNA from plants, performing reverse transcription, and conducting PCR using primers similar to those described above. Genomic DNA and cDNA may also be prepared by constructing a genomic DNA library or a cDNA library according to the standard method, and screening this library using a probe, for example, one synthesized based on the a nucleotide sequence of a DNA of the present invention (e. g. the sequence set forth in SEQ ID NO: 2) .

The DNA thus obtained may be easily sequenced using, for example, the "Sequencer Model 373" (ABI). DNAs of the present invention can be used, for example, in the preparation of recombinant proteins as described above. Furthermore, by expressing a DNA of the present invention within a plant, it is possible to obtain transformed plants having enhanced biosynthesis of a cell wall component and accelerated development/differentiation.

The present invention also provides molecules capable of suppressing the expression of the DNAs of this invention within plants. "Suppression of the expression of a DNA of this invention" includes the suppression of both gene transcription and translation into the protein. Moreover, not only complete inhibition, but also reduction of DNA expression is included.

As a method for suppressing the expression of a specific endogenous gene in plants, the anti-sense technique is widely used among those skilled in the art. The anti-sense effect in plant cells was first demonstrated by Ecker et al. by introducing anti-sense RNA by the electroporation technique using the transient gene expression method (J. R. Ecker and R. W. Davis, Proc. Natl. Acad. Sci. U.S.A. 83: 5372 (1986)). Since then, cases showing a reduction in target gene expression due to the expression of anti-sense RNA have been reported in tobacco and petunia plants (A. R. van der Krol et al., Nature 333: 866-869 (1988)). This method is now established as a means of suppressing gene expression in plants.

Several factors such as those described below are involved in the action of an anti-sense nucleic acid to suppress the expression of a target gene. These factors include: the inhibition of transcription initiation due to the formation of a triple strand; transcriptional suppression due to hybridization to a site in which a open loop structure has been formed locally by RNA polymerase; transcriptional inhibition due to hybridization to RNA whose biosynthesis is in progress; splicing suppression due to hybridization at the border of an intron and an exon; splicing suppression due to hybridization to the spliceosome-forming site; transport inhibition of mRNA from the nucleus to cytosol due to hybridization to the mRNA; splicing suppression due to hybridization at the capping site and poly(A) addition site; translation initiation suppression due to hybridization to the translation initiation factor-binding site; translational suppression due to hybridization to the ribosome-binding site in the vicinity of the initiation codon; suppression of polypeptide chain elongation due to hybridization to mRNA translational region and polysome-binding site; suppression of gene expression due to hybridization to the interaction site between nucleic acid and protein. These phenomena inhibit the processes of transcription, splicing or translation to suppress the expression of the target gene (Hirashima and Inoue, "Shin Seikagaku Jikken Kouza 2, Kakusan IV, Idenshi no Hukusei to Hatsugen", Nihon Seikagakukai eds., Tokyo Kagaku Doujin, pp. 319-347, 1993).

An anti-sense sequence used in the present invention may suppress the expression of a target gene by any of the above-described actions. As one embodiment, an anti-sense sequence designed to be complementary to the noncoding region in the 5'-end vicinity of the mRNA of a gene will be effective in inhibiting the translation of the gene. However, a sequence complementary to the coding region or noncoding region on the 3'-side can be also used. Thus, anti-sense DNAs used in the present invention also include DNA comprising an anti-sense sequence of not only the coding region, but also the noncoding region of the gene. Anti-sense DNA to be used is linked to the downstream of an appropriate promoter, and, preferably, a sequence containing a translational termination signal is linked to the 3'-side thereof. DNA thus prepared can be transformed into a desired plant by a method known in the art. The anti-sense DNA sequence is preferably complementary to the endogenous gene, or a portion thereof, of the plant to be transformed, but may need not be completely complementary as long as it is capable of effectively suppressing gene expression. The transcribed RNA has preferably 90% or more, most preferably 95% or more homology to the transcriptional product of a target gene. Sequence homology can be determined by the aforementioned searches.

To effectively suppress the expression of a target gene using an anti-sense sequence, the length of the anti-sense DNA should be at least 15 nucleotides or more, preferably 100 nucleotides or more, and even more preferably 500 nucleotides or more. Usually, the length of the anti-sense DNA to be used is less than 5 kb, preferably less than 2.5 kb.

It is also possible to carry out the expression suppression of an endogenous gene using a DNA coding for a ribozyme. "Ribozyme" refers to any catalytically active RNA molecule. There are ribozymes having a variety of activities, and, among them, studies on ribozymes as enzymes to cleave RNAs have enabled the design of ribozymes that aim at site-specific cleavage of RNAs. There are ribozymes having a size of 400 nucleotides or more, such as the group I intron ribozyme and M1 RNA contained in RNase P, and also those called hammerhead and hairpin ribozymes having an active domain comprising about 40 nucleotides (M. Koizumi and E. Ohtsuka, Protein, Nucleic acid, and Enzyme 35: 2191 (1990)).

For example, the self-splicing domain of hammerhead ribozymes cleaves the 3'-side of C15 in G13U14C15, and the formation of a base pair between U14 and A9 is thought to be important for the activity. It has been shown that the nucleotide at position 15 is spliced even by A or U, besides C (M. Koizumi et al., FEBS Lett. 228: 225 (1988)). If the substrate-binding site of a ribozyme is designed so as to become complementary to an RNA sequence adjacent to a target site, a restriction enzyme-like RNA splicing ribozyme that recognizes the sequence UC, UU, or UA in the target RNA can be made (M. Koizumi et al., FEBS Lett. 239: 285 (1988); M. Koizumi and E. Ohtsuka, Protein, Nucleic Acid, and Enzyme 35: 2191 (1990); M. Koizumi et al., Nucleic Acids Res. 17: 7059 (1989)). For example, a plurality of sites that can be targeted exist in the coding region of the *ACW2* gene (SEQ ID NO: 2).

The hairpin ribozyme is also useful for the purpose of the present invention. This ribozyme is found, for example, in the negative strand of the satellite RNA of the tobacco ringspot virus (J. M. Buzayan, Nature 323: 349 (1986)). It has been shown that this ribozyme too can be designed so as to catalyze target site-specific RNA cleavage (Y. Kikuchi and N. Sasaki, Nucleic Acids Res. 19: 6751 (1992); Y. Kikuchi, Chemistry and Biology 30: 112 (1992)).

A ribozyme designed to cleave a target site is linked to a promoter such as the 35S promoter of cauliflower mosaic virus and the transcription termination sequence so as to be transcribed in plant cells. However, when an extra sequence is attached to the 5'- and 3'-ends of the transcribed RNA, the ribozyme activity may sometimes be lost. In such a case, it is possible to arrange a different *cis*-acting trimming ribozyme on the 5'- and 3'-ends of the ribozyme portion so as to accurately excise only the ribozyme portion from the transcribed RNA containing the ribozyme (K. Taira et al., Protein Eng. 3: 733 (1990); A. M. Dzianott and J. J. Bujarski, Proc. Natl. Acad. Sci. U.S.A. 86: 4823 (1989); C. A. Grosshans and R. T. Cech, Nucleic Acids Res. 19: 3875 (1991); K. Taira et al., Nucleic Acids Res. 19: 5125 (1991)). Furthermore, to enhance the effect, it is also possible to arrange these constitutive units in tandem so that several sites within the target gene is cleaved (N. Yuyama et al., Biochem. Biophys. Res. Commun. 186: 1271 (1992)). The use of such ribozymes enables the site-specific cleavage of a transcriptional product of a target gene in the present invention to suppress gene expression.

Furthermore, endogenous gene expression can be suppressed also by co-suppression that results when a cell is transformed with a DNA having a sequence identical or analogous to that of a target gene. "Co-suppression" refers to a phenomenon that occurs when a gene having a sequence identical or analogous to a target endogenous gene is introduced into a plant by transformation resulting in the suppression of the expression of both the introduced exogenous gene and the target endogenous gene. The mechanism of co-suppression is not yet clear, but this phenomenon has been frequently observed in plants (Curr. Biol. 7: R793 (1997) : Curr. Biol. 6:810 (1996)). For example, to obtain a plant having the co-suppressed *ACW2* gene, the target plant is transformed with a vector DNA prepared so as to enable the expression of the *ACW2* gene or a DNA having a sequence analogous thereto, and a plant that has the characteristics of a acw2 mutant, for example, a plant in which cell wall biosynthesis is suppressed, is selected. Although the gene used in co-suppression need not be completely identical to the target gene, it has at least 70% or more, preferably 80% or more, even more preferably 90% or more (for example, 95% or more) sequence identity thereto. Sequence identity can be determined using the aforementioned search methods.

Furthermore, the suppression of endogenous gene expression in the present invention can be achieved also by transforming a plant with a gene having a dominant negative characteristic against the target gene. "DNA encoding a protein having a dominant negative characteristic" in the present invention refers to DNA encoding a protein which functions, upon the expression of the DNA, to eliminate or reduce the activity of the protein encoded by the endogenous gene that is intrinsic to the plant. Whether or not the DNA of interest is capable of eliminating or reducing the activity of the endogenous gene of this invention can be determined as aforementioned by its capability or inability to suppress cell wall biosynthesis and development/differentiation of the plant.

The present invention also provides a vector into which an above-described DNA of this invention or a DNA that suppresses the expression of a DNA of the present invention is inserted. Vectors of this invention include, besides the aforementioned vector used in the production of the recombinant protein, those expressing DNAs of this invention in plant cells to prepare transformed plants or DNAs that suppress the expression of DNAs of this invention. There is no particular limitation on the type of such vectors, as long as they contain a promoter sequence capable of initiating transcription in plant cells and a terminator sequence containing a polyadenylation site necessary for stabilizing the transcriptional product. Examples are plasmids "pBI121," "pBI221," "pBI101" (all from Clontech), and the like.

Vectors of the present invention may comprise a promoter to constantly or inductively express a protein of this invention. Promoters for constant expression are exemplified by the 35S promoter of cauliflower mosaic virus (Odell et al., Nature 313: 810 (1985)), the actin promoter of rice (Zhang et al., Plant Cell 3: 1155 (1991)), the ubiquitin promoter of corn (Cornejo et al., Plant Mol. Biol. 23: 567 (1993)), etc.

Furthermore, promoters for inductive expression are exemplified by promoters that are expressed by extrinsic factors such as infection and invasion of filamentous fungi, bacteria, and viruses, low temperature, high temperature, drought, ultraviolet irradiation, spraying of particular compounds, and the like. Such promoters are exemplified by the chitinase gene promoter of rice (Xu et al., Plant Mol. Biol. 30: 387 (1996)) and tobacco PR protein gene promoter (Ohshima et al., Plant Cell 2: 95 (1990)) expressed by the infection and invasion of filamentous fungi, bacteria and viruses, the "*lip 19*" gene promoter of rice induced by low temperature (Aguan et al., Mol. Gen Genet. 240: 1 (1993)), "*hsp 80*" and "*hsp 72"* gene promotors of rice induced by high temperature (Van Breusegem et al., Planta 193: 57 (1994)), "*rab 16"* gene promoter of *Arabidopsis thaliana* induced by dryness (Nundy et al., Proc. Natl. Acad. Sci. U.S.A. 87: 1406 (1990)), chalcone synthase gene promoter of parsley induced by ultraviolet irradiation (Schulze-Lefert et al., EMBO J. 8: 651 (1989)), alcohol dehydrogenase gene promoter of corn induced by anaerobic conditions (Walker et al., Proc. Natl. Acad. Sci. U.S.A. 84: 6624 (1987)) and so on. In addition, the chitinase gene promoter of rice and PR protein gene promoter of tobacco are induced also by specific compounds such as salicylic acid, and such, and the "*rab 16"* gene promoter is induced by the spraying of abcisic acid, a phytohormone.

Furthermore, the present invention provides transformed cells into which a vector of this invention has been introduced. Cells to which a vector of this invention is introduced include, besides the above-described cells used for the production of recombinant proteins, plant cells for preparing transformed plants. There is no particular limitation on the type of plant cells, and examples are cells of *Arabidopsis thaliana,* rice, corn, potato, tobacco, etc. Plant cells of this invention include, besides cultured cells, cells within the plant, and also protoplasts, shoot primordium, multiple shoots, hairy roots. Vectors can be introduced into plant cells by, for example, the method using Agrobacteria(Hood et al., Transgenic Res. 2: 218 (1993); Hiei et al., Plant J. 6: 271 (1994)), the electroporation method (Tada et al., Theor. Appl. Genet. 80: 475 (1990)), the polyethylene glycol method (Lazzeri et al., Theor. Appl. Genet. 81: 437 (1991)), the particle gun method (Sanford et al., J. Part. Sci. tech. 5: 27 (1987)), etc.

It is possible to regenerate plants by re-differentiating transformed plant cells. Methods of re-differentiation vary depending on the type of plant cells, but examples are the method of Fujimura et al. (Plant Tissue Culture Lett. 2: 74 (1995)) for rice, the methods of Shillito et al. (Bio/Technology 7: 581 (1989)) and Gorden-Kamm et al. (Plant Cell 2: 603 (1990)) for corn, the method of Visser et al. (Theor. Appl. Genet. 78: 594 (1989)) for potato, the method of Nagata and Takebe (Planta 99: 12 (1971)) for tobacco, that the method of Akama et al. (Plant Cell Reports 12: 7-11 (1992)) for *Arabidopsis thaliana,* and the method of Dohi et al. (Unexamined Published Japanese Patent Application No. Hei8-89113) for *Eucalyptus*.

Once a transformed plant to which a DNA of the present invention or a DNA that suppresses the expression of a DNA of the present invention has been integrated into the genome thereof, it is possible to obtain a progeny of the plant by sexual or asexual reproduction. It is also possible to obtain propagation material (such as seeds, fruits, spikes, tubers, tuberous roots, stubs, cali, protoplasts, etc.) from the plant or a progeny or clone thereof, and mass-produce the plant based on such. The present invention includes, plant cells to which a DNA of the present invention or a DNA that suppresses the expression of a DNA of the present invention has been introduced, plants containing the cells, progenyies and clones of the plants, as well as propagation material of the plants, progenies and clones.

In a transformed plant of the present invention, a cell wall component or development/differentiation may be changed compared to the normal plant by regulating the expression of a DNA of this invention. "Changes in a cell wall component" refers to, for example, quantitative and qualitative changes in a cell wall component such as cellulose. "Changes in development/differentiation" refers to, for example, changes in the cell division, cell number , and cell morphology. Through these changes, it is possible, for example, to achieve an increase in the supply efficiency of plants for fiber raw materials such as pulp, develop novel raw materials by regulating cell wall biosynthesis, increase the level of useful constituents of farm crops, increase the digestion/absorption efficiency of feed crops, increase the level of plant growth by enhancing the cell wall biosynthesis level and regulating development/differentiation, generate ornamental plants with novel aesthetic values due to altered cell morphologies that occur as a result of changes in cell wall components and development/differentiation, etc.

Furthermore, the present invention provides antibodies binding to above-described proteins of this invention, which includes polyclonal and monoclonal antibodies. Antibodies can be prepared by methods known to those skilled in the art (such as those described in "Molecular Cloning" (Maniatis et al., Cold Spring Harbor Laboratory Press). It is also possible to prepare an antibody of the present invention by immunizing suitable animals with not only a whole protein of this invention, but also with a partial peptide of a protein of this invention. The antibodies of the present invention may be used also for purifying and detecting a protein of this invention.

### Brief Description of the Drawings

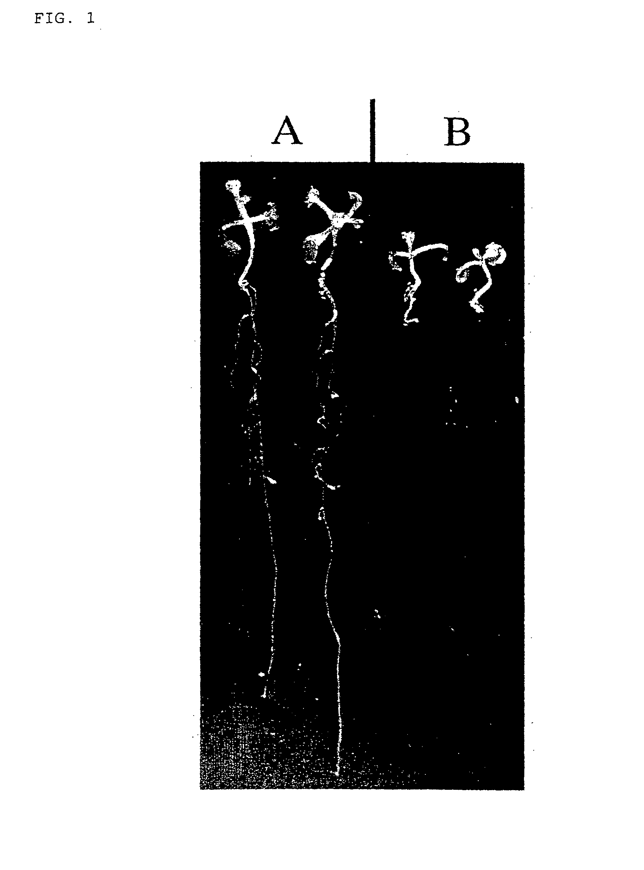

Fig. 1 represents optical micrographs showing morphologies of seedlings of acw2 mutants. A and B show the results with the wild type and mutant under the condition of 31°C (mutation temperature), respectively.

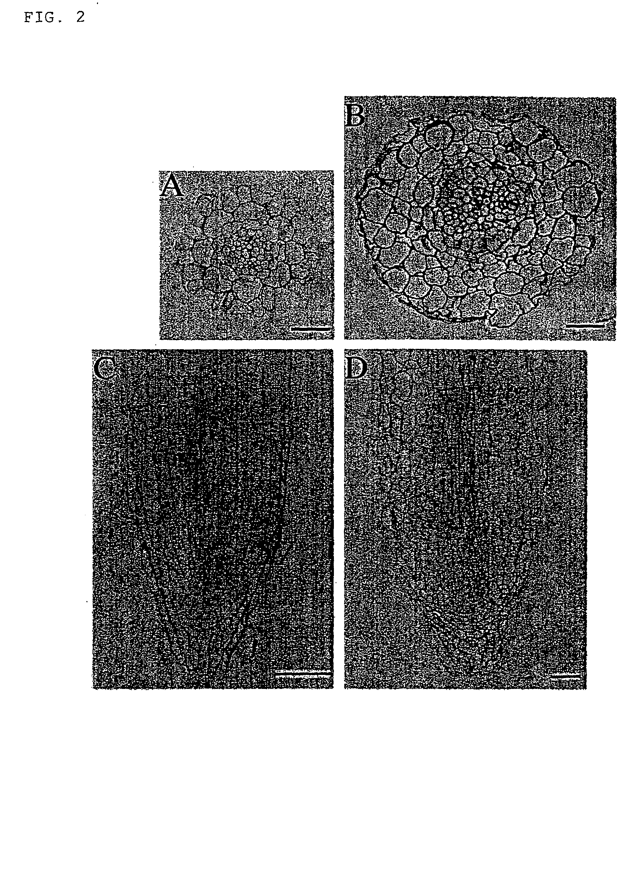

Fig. 2 represents a series of optical micrographs showing cross and vertical sectional slices of the acw2 mutant root. A and C respectively show cross and vertical sectional slices of the wild type, while B and D respectively show cross and vertical sectional slices of the mutant under the conditions of 31°C (mutation temperature).

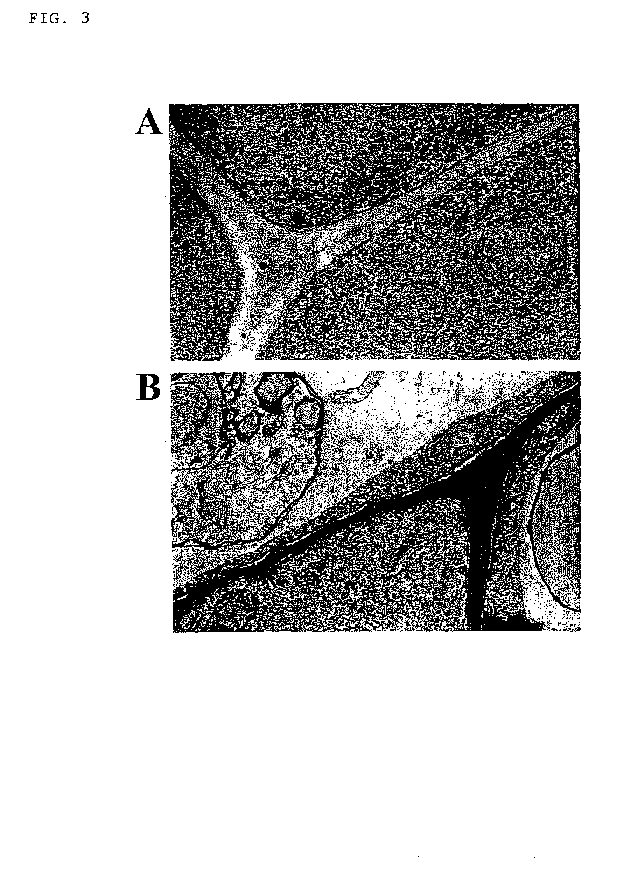

Fig. 3 represents electron micrographs showing the results of uranium acetate/lead citrate staining of an acw2 mutant root slice. A and B show the results with the wild type and mutant under the conditions of 31°C (mutation temperature), respectively.

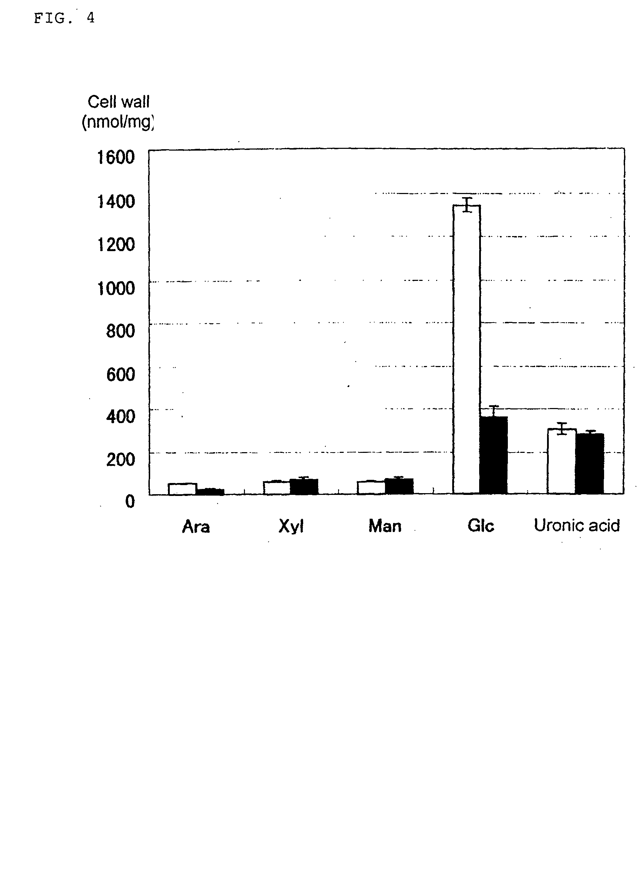

Fig. 4 represents a graph showing the analytical results of constitutive sugars in a cell wall TFA-insoluble fraction. White bars represent the results with the wild type, and black bars represent the results with the mutant under the conditions of 31°C (mutation temperature) . Ara stands for arabinose, Xyl xylose, Man mannose, and Glc glucose, respectively.

### Best Mode for Carrying out the Invention

Herein below, the present invention will be specifically described using Examples, however, it is not to be construed as being limited thereto. Herein, methods necessary for gene recombination in general such as cleavage and ligation of DNA, transformation of *Escherichia coli*, nucleotide sequencing, hybridization and the like were principally conducted as described in manuals attached to commercial reagents, equipments, and such used in the respective procedures, and laboratory manuals (such as "Molecular Cloning (Maniatis T. et al., Cold Spring Harbor Laboratory Press"). Furthermore, the raising of *Arabidopsis thaliana* using an agar medium or soil, mating, and preparation of genomic DNA thereof were basically carried out according to laboratory manuals (e.g. "Experimental Protocols of Model Plants (Shujunsha).

### [Example 1] Isolation of the ACW2 gene

First, to determine the locus of the *ACW2* gene on chromosomes, mapping was carried out using a molecular marker. Since an acw2 mutant has been found in the Columbia ecotype of *Arabidopsis thaliana*, the mutant was crossed with a different ecotype Landsberg *erecta* to harvest seeds of the first filial generation. These seeds were grown, and the plants were allowed to inbreed to obtain seeds of the F2 generation. These F2 generation seeds were germinated, and the genomic DNAs were extracted from the raised plants (389 plants) . For these genomic DNAs, the recombination values were calculated by PCR using, as markers, the known markers DFR and S0191, as well as 16L22, Sf2. Sr2 and JC20,2.2 prepared by the present inventors. More specifically, the genomic DNAs of the F2 generation were amplified by PCR using two PCR primers: "SEQ ID NO: 4/ 5-AGATCCTGAGGTGAGTTTTTC-3" and "SEQ ID NO: 5/ 5-TGTTACATGGCTTCATACCA-3" capable of amplifying DFR. PCR products were treated with the restriction enzyme BsaAI, and then examined by agarose gel electrophoresis. This molecular marker (DFR) has been known to indicate two restriction enzyme recognition sites in the Landsberg ecotype, and one recognition site in the Columbia ecotype. Calculations based on this information indicated recombination between the ACW2 gene and the marker on four chromosomes.

Next, the genomic DNAs of the F2 generation were amplified by PCR using a pair of PCR primers: "SEQ ID NO: 6/ 5-TGATGTTGATGGAGATGGTCA-3," and "SEQ ID NO: 7/ 5-CTCCACCAATCATGCAAATG-3" capable of amplifying S0191, and PCR products were examined by agarose gel electrophoresis. It is known that, with this molecular marker (S0191), the Landsberg ecotype shows a band at 156 bp, while the Columbia ecotype shows a band at 148 bp. Calculations based on this information indicated recombinations on 51 chromosomes between the *ACW2* gene and the marker.

To perform a more detailed mapping, a pair of PCR primers: "SEQ ID NO: 8/ 5-TGTATATTATAAATACTAAATTTATTGG-3" and "SEQ ID NO: 9/ 5-GAATATAGTCATTACATGAATAACC-3" capable of amplifying 16L22,Sf2.Sr2 were synthesized. Using these primers, the genomic DNAs of the F2 generation were amplified by PCR, and PCR products were examined by agarose gel electrophoresis. This molecular marker (16L22,Sf2.Sr2) shows polymorphisms between the Landbergh and Columbia ecotypes, and calculation based on this indicated recombination between the *ACW2* gene and the marker on one chromosome.

Then, a pair of PCR primers: "SEQ ID NO: 10/ 5-GCACATCATTGATCGGACTTTAC-3" and "SEQ ID NO: 11/ 5-CAACAAAGATCAAAACACTTCC-3" capable of amplifying JC20,2.2 were synthesized. Using these primers, the genomic DNAs of F2 generation were amplified by PCR, and PCR products were examined by agarose gel electrophoresis. With this molecular marker (JC20,2.2), it is known that the Landsberg ecotype shows a band at 87 bp, while the Columbia ecotype shows a band at 91 bp. Calculation based on this information showed recombination between the *ACW2* gene and the marker on one chromosome.

Next, a pair of PCR primers: "SEQ ID NO: 12/ 5-CAAACCGTCTATAACATTGTAC-3" and "SEQ ID NO: 13/ 5-GGATCTGGTTCACAAGTCTGTG-3" capable of amplifying JC20-1.1 were synthesized. Using these primers, the genomic DNAs of the F2 generation were amplified by PCR, and PCR products were examined by agarose gel electrophoresis. This molecular marker shows polymorphisms between the Landsberg and Columbia ecotypes, and calculation based on this fact revealed no recombination between the *ACW2* gene and the marker.

Thus, it was confirmed that the chromosomal region containing the *ACW2* gene is sandwiched between the two molecular markers 16L22,Sf2.Sr2 and JC20,2.2. Therefore, genomic DNA fragments containing each of the 16L22,Sf2.Sr2 and JC20,2.2 markers, were isolated and subjected to complementation testing. More specifically, ca. 10,000 clones of a TAC genomic DNA library (Liu, Y.-G. et al., Proc. Natl. Acad. Sci. U.S.A. 96: 6535-6540 (1999)) consisting of the genomic DNA of *Arabidopsis thaliana* Columbia ecotype was screened using 16L22,Sf2.Sr2 and JC20,2.2 as probes to obtain three clones (16L22, 19K1, and 11D4) with the former probe and one clone (22123) with the latter probe. These clones were transferred back into the acw2 mutant via an *Agrobacterium* (MP90 strain) by the Floral Dip method (Clough S. J., Bent A. F., "Floral dip: a simplified method for Agrobacterium-mediated transformation of *Arabidopsis thaliana",* Plant J. 16: 735-743 (1998)). As a result, only the 22I23 clone could complement the acw2 phenotype to the wild type. Therefore, it was revealed that the *ACW2* gene is contained in approximately the 30-kb region corresponding to the genomic DNA fragment contained in the 22I23 clone from which the region in which recombination was detected with the JC20,2.2 marker is subtracted.

A database search for nucleotide sequences in this region predicted the presence of five genes between the JC20,2.2 marker and the above-described DNA region. Comparison of nucleotide sequences of these five genes in the wild type with respect to those of the acw2 mutant revealed a mutation in the gene region corresponding to cDNA registered as a GTP-binding protein. Among the five genes, the mutation of the nucleotide sequence was detected only in the gene encoding this GTP-binding protein, and therefore, this gene proved to be the *ACW2* gene.

The full-length cDNA of the *ACW2* gene isolated from a λZAPIIcDNA library (STRATAGENE) was found to contain 15 introns. Analysis of the amino acid sequence encoded by the gene proved the identity as the dynamin-like protein ADL1 (Dombrowski, J. E. and Raikhel, N. V., "Isolation of a cDNA encoding a novel GTP-binding protein of *Arabidopsis thaliana",* Plant Mol. Biol. 28: 1121-1126 (1995)).

Dynamin-like proteins are one type of GTP-binding proteins broadly distributed in organisms such as yeast, mice, humans, plants, and such, and form a protein family. Dynamin-like protein homologues have been isolated from higher plants such as *Arabidopsis,* tobacco, soybean, and such, showing extremely high homology to each other. Dynamin-like proteins are characteristic in having highly homologous GTP-binding domains at their N-termini. The dynamin-like protein family is thought to function in association with endocytosis in animal cells. Although no definite function has been shown in plant cells, its involvement in vesicular fusion during cell plate formation and chloroplast formation has been suggested (Gu, X. and Verma, D. P. S., "Phragmoplastin, a dynamin-like protein associated with cell plate formation in plants", EMBO, J. 15: 695-704 (1996); Park, J. M. et al., "A dynamin-like protein in *Arabidopsis thaliana* is involved in biogenesis of thylakoid membranes", EMBO, J. 17: 859-867 (1998)). There has been no report on the involvement of ALD1 protein in the cell wall biosynthesis.

### [Example 2] Analysis of the cell wall of the acw2 mutant

A crude cell wall sample was prepared according to the method of Zablackis et al. (Zablackis, E. et al., "Characterization of cell-wall polysaccharides of *Arabidopsis thaliana* leaves", Plant Physiol. 107, 1129-1138 (1995)). The crude cell wall sample thus prepared was stored at -80°C, and a suitable amount was thawed prior to use for analysis.

An appropriate amount of the cell wall sample thus prepared was placed in a glass tube, 2 M trifluoroacetic acid (TFA) was added, and amorphous polysaccharides (hemicellulose/pectin) were hydrolyzed at 121°C for 1 h. The reaction mixture was left standing to cool down to room temperature, and then centrifuged to separate crystalline polysaccharides (cellulose) from the supernatant. Cellulose completely hydrolyzes in 72% H₂SO₄. For sugar quantification, the total sugar content was quantified by the phenol-sulfuric acid method (Dubois, M. et al., "Colorimetric method for determination of sugars and related substances", Anal. Biochem. 28, 350-356 (1956)), while uronic acid content was quantified by the m-hydroxybiphenyl method (Blumenkrantz, N. and Asboe-hansen, H. M., "New method for quantitative determination of uronic acids", Anal. Biochem. 54, 484-489 (1973)). As a result, it was revealed that the total sugar content in the cellulose fraction of the acw2 mutant was significantly reduced.

To examine the cause of the reduction in detail, constitutive sugar analysis was conducted using the alditol acetate method (York, W. S., et al., "Isolation and characterization of plant cell wall and plant cell components", Methods enzymol. 118, 3-40 (1986)). As a result, the glucose content (361.6 ± 50.8 nmol/mg cell wall) of the acw2 mutant was significantly reduced as compared to that (1342.5 ± 31.5 nmol/mg cell wall) of the wild type . Glucose in the TFA-insoluble fraction derives from cellulose, which proves that the acw2 mutant is a cellulose synthesis mutant.

From the above-described results, the present inventors showed a novel function of ACW2 protein (ADL1) that is involved in the biosynthesis of a main cell wall component, cellulose.

### Industrial Applicability

The present invention has provided a technique for producing plants having modified cell walls and altered development/differentiation by regulating the expression of the *ACW2* gene or genes analogous thereto. The modification of the cell wall and development/differentiation in plants can lead to, for example, an increase in the supply efficiency of plants for fiber raw material' such as pulp, the development of novel raw materials by regulating cell wall biosynthesis and development/differentiation, an increase in useful constituent levels of farm crops, an increase in the digestion/absorption efficiency of feed crops, an increase in the level of plant growth by enhancing the cell wall biosynthesis level and regulating development/differentiation, the production of ornamental plants with novel aesthetic values by altering cell morphologies associated with modification of cell wall components, and such. Therefore, such modifications are useful in the fields of agriculture and industry/horticulture. The ACW2 gene, genes analogous thereto, and molecules that suppress the expression of these genes (anti-sense DNAs, ribozymes, dominant negative molecules, antibodies, and the like) may be used as agents that modify a cell wall component or development/differentiation of plants.

## Claims

1. A DNA of any one of (a) to (d), wherein said DNA is used to modify a plant cell wall component or to regulate development/differentiation of a plant:
(a) a DNA encoding a protein comprising the amino acid sequence of SEQ ID NO: 1,
(b) a DNA comprising the nucleotide sequence of SEQ ID NO: 2,
(c) a DNA encoding a protein comprising the amino acid sequence of SEQ ID NO: 1 in which one or more amino acids have been substituted, deleted, or added, wherein said protein is functionally equivalent to the protein comprising the amino acid sequence of SEQ ID NO: 1, and
(d) a DNA that (i) is capable of hybridizing to the DNA comprising the nucleotide sequence of SEQ ID NO: 2 under stringent conditions, and (ii) encodes a protein that is functionally equivalent to the protein comprising the amino acid sequence of SEQ ID NO: 1.

2. A DNA coding for anti-sense RNA complementary to the transcriptional product of the DNA of claim 1, wherein said DNA is used for modifying a plant cell wall component or for regulating plant development/differentiation.

3. A DNA coding for RNA having a ribozyme activity to specifically cleave a transcriptional product of the DNA of claim 1, wherein said DNA is used for modifying a plant cell wall component or for regulating plant development/differentiation.

4. A DNA used for modifying a plant cell wall component or regulating plant development/differentiation, wherein said DNA (i) encodes an RNA that suppresses the expression of the DNA of claim 1 by co-suppression when it is expressed in plant cells, and (ii) has 90% or more homology to the DNA of claim 1.

5. A DNA used for modifying a plant cell wall component or regulating development/differentiation, wherein said DNA encodes a protein having a dominant negative characteristic against a plant cell endogenous protein encoded by the DNA of claim 1.

6. A vector comprising the DNA according to any one of claims 1 to 5.

7. A transformed cell retaining a DNA according to any one of claims 1 to 5, or the vector according to claim 6.

8. A protein encoded by the DNA according to claim 1.

9. A method for preparing the protein according to claim 8, comprising the steps of culturing transformed cells retaining the DNA according to claim 1, or a vector containing said DNA, and recovering the protein expressed from said transformed cells or culture supernatant thereof.

10. A transformed plant cell retaining the DNA according to any one of claims 1 to 5, or the vector according to claim 6.

11. A transformed plant comprising the transformed plant cell according to claim 10.

12. A transformed plant which is a progeny or a clone of the transformed plant according to claim 11.

13. The transformed plant according to claim 11 or 12, wherein a cell wall component has been modified.

14. The transformed plant according to claim 13, wherein the modification of the cell wall component is the alteration of the glucan content.

15. The transformed plant according to claim 13, wherein the modification of the cell wall component is the alteration of the cellulose content.

16. The transformed plant according to claim 11 or 12, wherein the development/differentiation has been modified.

17. The transformed plant according to claim 16, wherein the modification of development/differentiation is the alteration of cell division.

18. The transformed plant according to claim 16, wherein the modification of development/differentiation is the alteration of the number and size of cells.

19. A propagation material of the transformed plant according to any one of claims 11 to 18.

20. An antibody binding to the protein according to claim 8.

21. An agent for modifying a plant cell wall component or regulating plant development/differentiation including the DNA according to any one of claims 1 to 5, the vector according to claim 6, the protein according to claim 8, or the antibody according to claim 20.


SEQUENCE LISTING

<110> Oji Paper Co.,Ltd.

<120> MODIFICATION OF PLANT CELL WALL COMPONENTS AND METHOD
      FOR REGULATING PLANT DEVELOPMENT/DIFFERENTIATION

<130> 65 106 - EP

<140> EP 01/967 826
<141> 2001-09-25

<150> PCT/JP 01/08 301
<151> 2001-09-25

<150> JP 00/291 072
<151> 2000-09-25

<160> 13

<170> PatentIn Ver. 2.1

<210> 1
<211> 610
<212> PRT
<213> Arabidopsis thaliana

<400> 1
Met Glu Asn Leu Ile Ser Leu Val Asn Lys Ile Gln Arg Ala Cys Thr
  1               5                  10                  15

Ala Leu Gly Asp His Gly Asp Ser Ser Ala Leu Pro Thr Leu Trp Asp
             20                  25                  30

Ser Leu Pro Ala Ile Ala Val Val Gly Gly Gln Ser Ser Gly Lys Ser
         35                  40                  45

Ser Val Leu Glu Ser Ile Val Gly Lys Asp Phe Leu Pro Arg Gly Ser
     50                  55                  60

Gly Ile Val Thr Arg Arg Pro Leu Val Leu Gln Leu Gln Lys Ile Asp
 65                  70                  75                  80

Asp Gly Thr Arg Glu Tyr Ala Glu Phe Leu His Leu Pro Arg Lys Lys
                 85                  90                  95

Phe Thr Asp Phe Ala Ala Val Arg Lys Glu Ile Gln Asp Glu Thr Asp
            100                 105                 110

Arg Glu Thr Gly Arg Ser Lys Ala Ile Ser Ser Val Pro Ile His Leu
        115                 120                 125

Ser Ile Tyr Ser Pro Asn Val Val Asn Leu Thr Leu Ile Asp Leu Pro
    130                 135                 140

Gly Leu Thr Lys Val Ala Val Asp Gly Gln Ser Asp Ser Ile Val Lys
145                 150                 155                 160

Asp Ile Glu Asn Met Val Arg Ser Tyr Ile Glu Lys Pro Asn Cys Ile
                165                 170                 175

Ile Leu Ala Ile Ser Pro Ala Asn Gln Asp Leu Ala Thr Ser Asp Ala
            180                 185                 190

Ile Lys Ile Ser Arg Glu Val Asp Pro Ser Gly Asp Arg Thr Phe Gly
        195                 200                 205

Val Leu Thr Lys Ile Asp Leu Met Asp Lys Gly Thr Asp Ala Val Glu
    210                 215                 220

Ile Leu Glu Gly Arg Ser Phe Lys Leu Lys Tyr Pro Trp Val Gly Val
225                 230                 235                 240

Val Asn Arg Ser Gln Ala Asp Ile Asn Lys Asn Val Asp Met Ile Ala
                245                 250                 255

Ala Arg Lys Arg Glu Arg Glu Tyr Phe Ser Asn Thr Thr Glu Tyr Arg
            260                 265                 270

His Leu Ala Asn Lys Met Gly Ser Glu His Leu Ala Lys Met Leu Ser
        275                 280                 285

Lys His Leu Glu Arg Val Ile Lys Ser Arg Ile Pro Gly Ile Gln Ser
    290                 295                 300

Leu Ile Asn Lys Thr Val Leu Glu Leu Glu Thr Glu Leu Ser Arg Leu
305                 310                 315                 320

Gly Lys Pro Ile Ala Ala Asp Ala Gly Gly Lys Leu Tyr Ser Ile Met
                325                 330                 335

Glu Ile Cys Arg Leu Phe Asp Gln Ile Phe Lys Glu His Leu Asp Gly
            340                 345                 350

Val Arg Ala Gly Gly Glu Lys Val Tyr Asn Val Phe Asp Asn Gln Leu
        355                 360                 365

Pro Ala Ala Leu Lys Arg Leu Gln Phe Asp Lys Gln Leu Ala Met Asp
    370                 375                 380

Asn Ile Arg Lys Leu Val Thr Glu Ala Asp Gly Tyr Gln Pro His Leu
385                 390                 395                 400

Ile Ala Pro Glu Gln Gly Tyr Arg Arg Leu Ile Glu Ser Ser Ile Val
                405                 410                 415

Ser Ile Arg Gly Pro Ala Glu Ala Ser Val Asp Thr Val His Ala Ile
            420                 425                 430

Leu Lys Asp Leu Val His Lys Ser Val Asn Glu Thr Val Glu Leu Lys
        435                 440                 445

Gln Tyr Pro Ala Leu Arg Val Glu Val Thr Asn Ala Ala Ile Glu Ser
    450                 455                 460

Leu Asp Lys Met Arg Glu Gly Ser Lys Lys Ala Thr Leu Gln Leu Val
465                 470                 475                 480

Asp Met Glu Cys Ser Tyr Leu Thr Val Asp Phe Phe Arg Lys Leu Pro
                485                 490                 495

Gln Asp Val Glu Lys Gly Gly Asn Pro Thr His Ser Ile Phe Asp Arg

500                 505                 510

Tyr Asn Asp Ser Tyr Leu Arg Arg Ile Gly Ser Asn Val Leu Ser Tyr
        515                 520                 525

Val Asn Met Val Cys Ala Gly Leu Arg Asn Ser Ile Pro Lys Ser Ile
    530                 535                 540

Val Tyr Cys Gln Val Arg Glu Ala Lys Arg Ser Leu Leu Asp His Phe
545                 550                 555                 560

Phe Ala Glu Leu Gly Thr Met Asp Met Lys Arg Leu Ser Ser Leu Leu
                565                 570                 575

Asn Glu Asp Pro Ala Ile Met Glu Arg Arg Ser Ala Ile Ser Lys Arg
            580                 585                 590

Leu Glu Leu Tyr Arg Ala Ala Gln Ser Glu Ile Asp Ala Val Ala Trp
        595                 600                 605

Ser Lys
    610

<210> 2
<211> 1830
<212> DNA
<213> Arabidopsis thaliana

<400> 2
atggaaaatc tgatctctct ggttaacaag atacagagag cttgcacggc tttaggagac 60
catggagact ccagcgcttt acctactctt tgggattcct tgcctgcgat cgccgtcgtt 120
ggtggtcaga gctcagggaa gtcttcagtc ctggagagca tcgtgggaaa ggacttttta 180
ccccgtggat ctggcattgt tactcgaagg ccccttgtct tacagttgca aaagatcgat 240
gatggaaccc gggagtatgc agagtttctt cacctcccga ggaaaaagtt tactgatttt 300
gctgctgtga ggaaggagat tcaagatgag actgacagag agactggacg cagcaaggct 360
atttctagtg ttcccattca ccttagcata tactctccca atgttgtcaa cttgacactg 420
atagatcttc cagggcttac aaaagttgct gttgatggac aatctgatag tatagtgaag 480
gacattgaaa acatggttcg gtcctacatt gaaaagccca actgcatcat tttggcaatc 540
tcacctgcaa accaagatct tgctacctca gatgcaatta aaatttcccg tgaggttgat 600
ccatcggggg acagaacatt tggtgtcttg acaaagattg atcttatgga caaggggacg 660
gatgcagtgg aaattctgga agggagatct tttaaactta aatatccgtg ggttggtgtc 720
gtcaaccgtt cccaagcaga tattaacaag aatgtcgaca tgattgcggc tcggaaaaga 780
gagagggagt acttttccaa tactactgag tataggcacc ttgctaataa aatgggttcc 840
gagcatttgg caaagatgct ctccaagcat ctagaacgtg tgatcaagtc gagaattcct 900
ggcattcagt cacttattaa caaaacagta ttagagctgg aaactgaact aagtcgcctt 960
ggaaagccta ttgcagctga tgcagggggg aagttgtact caataatgga gatatgtcgg 1020
ctttttgatc aaatattcaa agagcatctt gatggagtgc gtgctggtgg tgaaaaagtg 1080
tacaacgtgt ttgataacca gcttcctgcg gctctgaaga gactccaatt tgacaagcag 1140
ctagcgatgg acaacatccg gaagctggtc actgaggctg atggttacca gcctcacttg 1200
attgctcctg agcaaggtta ccgtcgtctc attgagtctt ctattgtctc catcagaggc 1260
cctgctgaag catctgttga caccgttcat gctatcttaa aggatctggt tcacaagtct 1320
gtgaatgaaa ctgtggaact aaaacaatac ccagctctga gagtggaggt gacaaatgcg 1380
gcgatagagt cgctggataa aatgcgggaa ggaagtaaga aagcaacact gcagctggtt 1440
gacatggagt gcagttacct cactgttgat ttcttcagga aacttcccca ggatgttgag 1500
aagggtggta accccacaca ctccattttc gaccgctaca acgattccta tctcagacga 1560
atcggatcca atgttttgtc ttacgtgaac atggtctgtg ctggcctgcg gaattcaatc 1620
cccaagtcca tcgtatactg ccaagtccga gaagcgaagc gcagtctcct cgaccatttc 1680
tttgcggagc tcggtaccat ggatatgaag aggctctcgt cgctattgaa cgaagatcca 1740
gcaatcatgg agagacgcag tgccatctca aagcggctag aattgtatcg agcagcccaa 1800
tccgagatcg atgctgttgc ttggtccaag                                  1830

<210> 3
<211> 8800
<212> DNA
<213> Arabidopsis thaliana

<220>
<223> genomic DNA

<220>
<221> exon
<222> (4032)..(4160)

<220>
<221> intron
<222> (4161)..(4527)

<220>
<221> exon
<222> (4528)..(4590)

<220>
<221> intron
<222> (4591)..(4686)

<220>
<221> exon
<222> (4687)..(4794)

<220>
<221> intron
<222> (4795)..(4891)

<220>
<221> exon
<222> (4882)..(4983)

<220>
<221> intron
<222> (4984)..(5067)

<220>
<221> exon
<222> (5068)..(5118)

<220>
<221> intron
<222> (5119)..(5243)

<220>
<221> exon
<222> (5244)..(5306)

<220>
<221> intron
<222> (5307)..(5479)

<220>
<221> exon
<222> (5480)..(5569)

<220>
<221> intron
<222> (5570)..(5664)

<220>
<221> exon
<222> (5665)..(5730)

<220>
<221> intron
<222> (5731)..(5927)

<220>
<221> exon
<222> (5928)..(6122)

<220>
<221> intron
<222> (6123)..(6374)

<220>
<221> exon
<222> (6375)..(6494)

<220>
<221> intron
<222> (6495)..(6562)

<220>
<221> exon
<222> (6563)..(6634)

<220>
<221> intron
<222> (6635)..(6773)

<220>
<221> exon
<222> (6774)..(6998)

<220>
<221> intron
<222> (6999)..(7102)

<220>
<221> exon
<222> (7103)..(7153)

<220>
<221> intron
<222> (7154)..(7251)

<220>
<221> exon
<222> (7252)..(7479)

<220>
<221> intron
<222> (7480)..(7552)

<220>
<221> exon
<222> (7553)..(7693)

<220>
<221> intron
<222> (7694)..(7778)

<220>
<221> exon
<222> (7779)..(7907)

<400> 3
caattctttc gtctgtcact tcagacaaag atataaatac agatccaaca aggcaaatag 60
tagtgaacaa agtttgcttc cttaagcaac ttttctaatc agccattttg tctatcaaat 120
gaaaagcaga gactcagatt aagtataacg agcagcacca agcgttttat gttaccagta 180
atacacatac tatgcattgg gggacaagac attcatagag tctatataag tatcctaaca 240
attcaagtaa aaacaaaaca ggtattgcat tctaataaag ataacaagag gaaaaaacca 300
aagaagagaa ggcggtttag caggcagctg atgatccggt ttactctcaa tcatcctgtc 360
aaacaaagta tgtttctttt tactgactca cacgtgacac gattattcca gtgatgactt 420
acaattcaaa ctcgtcttca agcttcagag cctcagcagc agcctcttct tcctcatcgt 480
caacaacaaa gtacgggtta tgcacctctg gcctgaactt gtagccagta aacagataga 540
aggcaagtgt ggctaactcg ctggcaacaa cactagtcca catatacttg taagaggtaa 600
tggtctccag tgcatacaca acaacacgag tgaagtagat gtagcagata accacaatgt 660
agtactgcct aaacagagtt aacttaacga ggttcacagc agccttcccg tcagtcttag 720
ccgcctcacg caagtttttg atggaccaga caatgggaaa cagaacagca cagcaacaga 780
cgacatcaac aagcaaaaag atctgtttcc acgtgaccca atcttggcca tacggtccag 840
tctcatcgat aaccacctga gcaaagttag cgacaacctg gagaggaatg acaatcatca 900
agaccttctt ttccttgtcc tgcaagtagg gcttcaagaa agaccaaccg gtgccgatca 960
agacgatcaa agtgaaaaga gtgataccct taaggaaatt gaaaatgtag aacaagacat 1020
cccaaccatg agcagttcca gttttcttaa tgtacgactt atcctcagcc tcacaaagaa 1080
ggttcaaagc tttcaagaca acaactccca acatgaagaa atggattgcg aaaacagtga 1140
gacgtttctt atagagaacg tagatccacg tggcggcgag agagaaatag ataacagaga 1200
ataagaaata gaccttagga agaacagttc ttccagcaga gagataatca cgacctccct 1260
ttttaccttc aaggttatac atagcggatc taacatccat actgattttg acctgttgga 1320
gacaattagc gaagacgaga gagtactgat ccgcatcgtt ctcggtgaat acggtggaga 1380
atctgctctt atctcctccc ttgagattgt tgaaggtgaa cacatgctta acgagatctg 1440
attgtagagc gcacgtgatc tcttcttctt caagctgttg gatcacatga acccaagcgt 1500
ctctagtgca gagaaagaag cccactttgg agagatcgag atcgggattg ctgttggaaa 1560
gccagatctt ggaagcgtca agctcaagac gaccgctgtg agtgaaaccg aattcgtcta 1620
aagggatgat tggacgatca tcggatcgga tctctgattt acgaatctca gccgaagcta 1680
tggagatgga gatcaagaga aggataagga tcgatgagaa gcgtgaagag agatccatgg 1740
gaactagggt ttcgttcttc gacttcacag agagattaat ctgatctcag atgtgagatc 1800
caaagcaaaa tcaatccaac ttgttcttct tccgatgaaa acggttgtgt gtgaccaacc 1860
aaattaaatt agtctttttt ttctttctaa gaaacaaaat aaataccatt attttattag 1920
caacaagaga acccattttc cttcatcatt aagggaataa accatctttg acggattagt 1980
tgatttctag aaacaaaaat ttcaccaatt caccgattcc aaccgaatgg ccggcgtttc 2040
cagtgtattt gaaggagttg tagagtgata gggaagaatt cataaatttc tctttatctt 2100
taatttctcg tagtgcaaat gtaaaagcgg ataagttggc acgaaagatt cgtaccgtac 2160
cgcatcatgt cacgtatgta aacaatatct ctcgggattg gatcttttga gttcaaattt 2220
atatcggttg acaaaaaaaa aaaaaaaaaa aaaaggaaga aattaaagta ttgatgaatt 2280
tcgttttgaa ttggtcggaa attggatgta taagttgaga acaaattatc catcaattta 2340
aatcataaaa tgtttttccg aatgactttt ttttggagat gtattatttc taaatgtttt 2400
tacggtttct ttagatctcg ttcagattgt atcaacaagc gcgaaacctt cattaaacat 2460
aaatggtata caaagaaagc ttaaatacta aaccggagag atattatcta tttataatag 2520
attggtttga ctgtttgagt gtctatggtc taactcttgt cctctctaca caacccgaca 2580
ctaaaccaac atcttccact cacatatgac gaaaaacatc agaaagtagt agaagagtac 2640
tcttagagat tttgttcaag aatcgcaatt cgaaaaagtt acactgcaaa taaaccgtgt 2700
gacccgtgca agtatatgtg cttttggaag ctaaattcat gcagctgcta cggagacata 2760
cagtctacat acctaaatat attacatagc atgtctcact ccaacctcat ttgtatttca 2820
tattagtctc aacatattcc cataaagcat atgttaaacc tattttacat cacgttttca 2880

ctaacttcaa aattctaatt tgagggatag aagttggtca catgatacat atttgttaac 2940
gtatgaacat cataaaatct aacaattgat acaagagtat gtaggataca aagttacacg 3000
ttgtgtttgt ctcaaacaac attagtaaca ttttttaaaa aaattgtgta gacaaacaca 3060
acattagtaa catttaaaaa aaaaaatgtg aaccggttta ttttttcacc acttagctgg 3120
aaaataaata acgtgtattt gcgtaaacaa tacataaggt ttaaaaatgt gttagtgcta 3180
tgttttatac atttctaaaa agcgtttgga tctcaccaac caaacatagt atttgccaaa 3240
atgcaaacgc tatctaaaat cgatgtgaca ttactagtca gatttagtgg gtttgttcta 3300
gaaaagatct ttattttata cattcaaatt ttcaaaacta cagttatttt gttgtttagt 3360
gtcatccttt tgaataaaca atcataatcc ttaacgtctc ttatcttttt tttgaatcct 3420
caatctatgg accgtttgaa ctgcaatcgt aatgaggatg ttcgtggtcc acatgaaatc 3480
actttactct actaataagt gatagttttt ttattcgaat tttaactcgt tacattcttt 3540
tacaagaaaa tcgaccttgt tatatttatt tatatggttg gtcatgcatg caatagtatt 3600
atcattactt tacctggtta gagagatgtg gaatattaag ggattggttg aatatatacg 3660
acacacctcg tcgtttttat tttgtttttg gtcaactcct cgtcgtcttt gaattctcga 3720
aataaacacc atgccgtttc aaatttcttt tggaaaagaa aagaccctgt attggctttt 3780
atataaaccg ctttcttccc ctttactttt tcccaacact agcaagcaaa aattcttcat 3840
cgactcaaat tcaaaaactc atctctctct atctctattt ctctggttcc atagctcacc 3900
gtcgcatcgc agatctactc cttccgcaat aaattttacc ggcggaggta tcagatctcg 3960
ccgatctgtt gtagcagcta ctgtattttg ggcttctcat ttgatattgg ggaaacgagg 4020
agtagaggac gatggaaaat ctgatctctc tggttaacaa gatacagaga gcttgcacgg 4080
ctttaggaga ccatggagac tccagcgctt tacctactct ttgggattcc ttgcctgcga 4140
tcgccgtcgt tggtggtcag gtttgcatct cgatctcctt ccccttccat tatatgcttg 4200
acgatctatc tcacgattcg ttcgtttttt ttgcgattcg gaattgatat tgagctgagg 4260
tattcggata aatttgccct aaacttcgtt gtttttgctt atcaacatcc ggatcgtgag 4320
gaattatagt ttaatagctt ctactatgaa ctagctttgc aagcaattgg tattgaggaa 4380
tgttgacttg gctttgactt ggatattttt tcactagttg gtcttgaacc atttaattta 4440
agctacgatt gttgtggaga ggggtttctg tactttgata attctatgtg gcattaagtt 4500
tatgttgtat ccttattgtg ttgacagagc tcagggaagt cttcagtcct ggagagcatc 4560
gtgggaaagg actttttacc ccgtggatct ggtaaataca gtgttaacac ttcttactta 4620
gttttaatta tgaaattgct tatttccttg agtaatgaca atatgcattt gcggtaacta 4680
tttgtaggca ttgttactcg aaggcccctt gtcttacagt tgcaaaagat cgatgatgga 4740
acccgggagt atgcagagtt tcttcacctc ccgaggaaaa agtttactga ttttggtggg 4800
tttcggctat ctcaatttgc agttcaagtt tcaccttctc ctgtattcca ttgatatttg 4860
attaatttaa tcacctctat agctgctgtg aggaaggaga ttcaagatga gactgacaga 4920
gagactggac gcagcaaggc tatttctagt gttcccattc accttagcat atactctccc 4980
aatggtaagg acaacccaaa atctctttcc agttgtatgt gctttttgtt cattctctaa 5040
ttttttatcc tctttactat gtgtgcagtt gtcaacttga cactgataga tcttccaggg 5100
cttacaaaag ttgctgttgg taagttacag tagagcaaga agttttggtc tattttttct 5160
agcgatgaag catgaggcaa tagactttat tctctaccat acataatttg gcatgacacg 5220
acaaatttct tttggttgat gcagatggac aatctgatag tatagtgaag gacattgaaa 5280
acatggttcg gtcctacatt gaaaaggtaa atgaaaatgc ttactcccag accctttaat 5340
gttttataga tagctatcgc cagtttaaat tagattcggt aatatataag tattctccct 5400
atatttgtta tgttttcgta gcgccaaatg agttgacact attctgtttt tttttttatt 5460
taattaaatt tccttatagc ccaactgcat cattttggca atctcacctg caaaccaaga 5520
tcttgctacc tcagatgcaa ttaaaatttc ccgtgaggtt gatccatcgg gtgagcattt 5580
tacaaacaat tcatttttcc acctaagaga cccaacacat tattctgtca tcctatgtct 5640
cttataatct tggtgatctt cacaggggac agaacatttg gtgtcttgac aaagattgat 5700
cttatggaca aggggacgga tgcagtggaa gtaagttgaa ttcccagttt atagaacatc 5760
tgtactagaa tttcgaatta ctgtgaataa aatgtgttgt aattagtcta tgatatgatg 5820
ttggagctat aaaatgctaa tttgtgtagt aacatcataa tacttatgtt ccctcattaa 5880
tgtttattgc gtgcaccgct acttaccaaa tgatattttt tgctcagatt ctggaaggga 5940
gatcttttaa acttaaatat ccgtgggttg gtgtcgtcaa ccgttcccaa gcagatatta 6000
acaagaatgt cgacatgatt gcggctcgga aaagagagag ggagtacttt tccaatacta 6060
ctgagtatag gcaccttgct aataaaatgg gttccgagca tttggcaaag atgctctcca 6120
aggtacgtgc tttcacgagg ctaggggtag ttttcttcac caggttcttt ataataagta 6180
agttggcgtt gtgaaatttg ttaggagtgg taaataagag tttcagggcc agtgttttca 6240
cgattgctgc taattttctt agcagccgac agaaaatact aagaaactac gaagttatgg 6300
caatgcttag cacgtttctc tgtataaaac catcaggaca gagcctaatt gcaacaattt 6360
tatttgttgc gcagcatcta gaacgtgtga tcaagtcgag aattcctggc attcagtcac 6420
ttattaacaa aacagtatta gagctggaaa ctgaactaag tcgccttgga aagcctattg 6480
cagctgatgc aggggtaaat tgtgtattct tttatttttg attgaattgc gctatcaaga 6540

tttacttaat gttctgatgc aggggaagtt gtactcaata atggagatat gtcggctttt 6600
tgatcaaata ttcaaagagc atcttgatgg agtgtacgtt ttgactcttt ttacttcaaa 6660
tgtttaagat tacaccatga gttgttatag cgtctgagtt gatatttcta ctcttgacct 6720
tctgaaacta caaaaggaaa attgtaattt aagactatgg ttgattacac aggcgtgctg 6780
gtggtgaaaa agtgtacaac gtgtttgata accagcttcc tgcggctctg aagagactcc 6840
aatttgacaa gcagctagcg atggacaaca tccggaagct ggtcactgag gctgatggtt 6900
accagcctca cttgattgct cctgagcaag gttaccgtcg tctcattgag tcttctattg 6960
tctccatcag aggccctgct gaagcatctg ttgacaccgt atgaactctt tactttcatc 7020
tctaaaaaga tgtatcaaaa acctaacaca aaacaataag cttctctctc cgtttttttt 7080
tttttctatg gttaaatatt aggttcatgc tatcttaaag gatctggttc acaagtctgt 7140
gaatgaaact gtggtacgtt ctttttcttt ttagtaatat atatatatat atatatatct 7200
ccaacaacta gtacttttgt acaatgttat agacggtttg aacatgtgca ggaactaaaa 7260
caatacccag ctctgagagt ggaggtgaca aatgcggcga tagagtcgct ggataaaatg 7320
cgggaaggaa gtaagaaagc aacactgcag ctggttgaca tggagtgcag ttacctcact 7380
gttgatttct tcaggaaact tccccaggat gttgagaagg gtggtaaccc cacacactcc 7440
attttcgacc gctacaacga ttcctatctc agacgaatcg gtaaggttaa cccatataaa 7500
taaataatta tctggtaccg tatatctcac aaattttaga catttggcaa caggatccaa 7560
tgttttgtct tacgtgaaca tggtctgtgc tggcctgcgg aattcaatcc ccaagtccat 7620
cgtatactgc caagtccgag aagcgaagcg cagtctcctc gaccatttct ttgcggagct 7680
cggtaccatg gatgtaagtg ctctctgcct tttttcttaa cctaaagaag tgtcattcgg 7740
gtaaacactg agtcaactaa tatggtgata tccaacagat gaagaggctc tcgtcgctat 7800
tgaacgaaga tccagcaatc atggagagac gcagtgccat ctcaaagcgg ctagaattgt 7860
atcgagcagc ccaatccgag atcgatgctg ttgcttggtc caagtgatac cggcatgtca 7920
tgtccactgt tttgctcggt tctggtcggt gtggctcaga ctcggagcag agatttaggg 7980
tctgtaattt gtataagatg atcttcccga taccatgcag tatcgtttta tataacatcc 8040
acattgtttg tcctacctct atgttttttg tccatcaccc gatatgttac gtatcgtttt 8100
ataacatcca cattgtttgt cctgcctcta atgttttttg tccatcaccc gatatgttac 8160
gtatctttta taacatccac atagattaga ttttctctat tccctttatt taattactat 8220
aaaatgtatt tttcgcatat gataattttt ctgtaacaat agtataatat taaacattct 8280
acccaaaaaa agagtataat atttaaatat gattaccaat aagaaaaaaa ttctaatagt 8340
ttaaaataaa aatttgtaat gtactctttt ttattttgtt ttgcgtttct gaatttgtgg 8400
ccattatctt ctcacactct cttctcttag ctcacagagg aaagaaaaat ggcgaattcc 8460
atttcatcaa tatctctgcc tcgatgtttc atcttcaaca atggtagtca caaatcaagg 8520
ccatggccaa gctcaagcag tttctttctc aacaaatcat caaagcatca tcctcatcca 8580
ctactctctc tttcttcttc gccgtcttcc gtcgtagaaa ctgataatga cgacgacaac 8640
gacctcactt tcaggtaccc ttttgcttct cttcatcgat tccattggaa agcttaaacc 8700
tttctcagct aatccaattt cgttgacatt aaggatttta gagttagtga tagattaggg 8760
attgcgtaaa tgatgaaatc atagagactc ttttctgctg 8800

<210> 4
<211> 21
<212> DNA
<213> Artificial Sequence

<220>
<223> Description of Artificial Sequence: an artificially
      synthesized primer sequence

<400> 4
agatcctgag gtgagttttt c 21

<210> 5
<211> 20
<212> DNA
<213> Artificial Sequence

<220>
<223> Description of Artificial Sequence: an artificially
      synthesized primer sequence

<400> 5
tgttacatgg cttcatacca                                                    20


<210> 6
<211> 21
<212> DNA
<213> Artificial Sequence

<220>
<223> Description of Artificial Sequence: an artificially
      synthesized primer sequence

<400> 6
tgatgttgat ggagatggtc a                                                  21


<210> 7
<211> 20
<212> DNA
<213> Artificial Sequence

<220>
<223> Description of Artificial Sequence: an artificially
      synthesized primer sequence

<400> 7
ctccaccaat catgcaaatg                                                    20


<210> 8
<211> 28
<212> DNA
<213> Artificial Sequence

<220>
<223> Description of Artificial Sequence: an artificially
      synthesized primer sequence

<400> 8
tgtatattat aaatactaaa tttattgg                                           28


<210> 9
<211> 25
<212> DNA
<213> Artificial Sequence

<220>
<223> Description of Artificial Sequence: an artificially
      synthesized primer sequence

<400> 9
gaatatagtc attacatgaa taacc                                              25


<210> 10
<211> 23
<212> DNA
<213> Artificial Sequence

<220>
<223> Description of Artificial Sequence: an artificially
      synthesized primer sequence

<400> 10
gcacatcatt gatcggactt tac                                              23


<210> 11
<211> 22
<212> DNA
<213> Artificial Sequence

<220>
<223> Description of Artificial Sequence: an artificially
      synthesized primer sequence

<400> 11
caacaaagat caaaacactt cc                                               22


<210> 12
<211> 22
<212> DNA
<213> Artificial Sequence

<220>
<223> Description of Artificial Sequence: an artificially
      synthesized primer sequence

<400> 12
caaaccgtct ataacattgt ac                                               22


<210> 13
<211> 22
<212> DNA
<213> Artificial Sequence

<220>
<223> Description of Artificial Sequence: an artificially
      synthesized primer sequence

<400> 13
ggatctggtt cacaagtctg tg                                               22